(19) Europäisches Patentamt
European Patent Office
Office européen des brevets

(11) Numéro de publication : **0 338 946 B1**

(12) # FASCICULE DE BREVET EUROPEEN

(45) Date de publication du fascicule du brevet : **01.07.92 Bulletin 92/27**

(51) Int. Cl.$^5$ : **A23L 1/236,** A23L 1/03, C07C 233/04, A61K 7/16, A61K 47/00

(21) Numéro de dépôt : **89420135.9**

(22) Date de dépôt : **14.04.89**

(54) **Agents édulcorants dérivant des acides L-aspartique et L-glutamique N-hydrocarbonés.**

(30) Priorité : **22.04.88 FR 8805644**

(43) Date de publication de la demande : **25.10.89 Bulletin 89/43**

(45) Mention de la délivrance du brevet : **01.07.92 Bulletin 92/27**

(84) Etats contractants désignés : **AT BE CH DE FR GB IT LI LU NL SE**

(56) Documents cités :
**EP-A- 0 107 597**
**EP-A- 0 242 896**
**US-A- 3 725 453**
**Patent Abstracts of Japan, vol. 11, no. 361 (C-459)(2808), 25 novembre 1981; & JP-A-62 132 847 (Ajinomoto Co. Inc)16.06.1987**

(73) Titulaire : **NOFRE, Claude**
**119, Cours Albert Thomas**
**F-69003 Lyon (FR)**
Titulaire : **TINTI, Jean-Marie**
**5, impasse de la Drelatière**
**F-69680 Chassieu (FR)**

(72) Inventeur : **Nofre, Claude**
**119 Cours Albert Thomas**
**F-69003 Lyon (FR)**
Inventeur : **Tinti, Jean Marie**
**5, avenue de Grenoble**
**F-69330 Meyzieu (FR)**

(74) Mandataire : **Hubert, Philippe et al**
**Cabinet Beau de Loménie 55, rue d'Amsterdam**
**F-75008 Paris (FR)**

**EP 0 338 946 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux agents édulcorants utiles, notamment, pour édulcorer les aliments, les boissons, les confiseries, les pâtisseries, les chewing-gums, les produits d'hygiène, les cosmétiques, les articles de toilette, les produits pharmaceutiques et vétérinaires, et leurs équivalents.

Dans les brevets US-A-3.725.453 et 3.775.460 il a été décrit des agents édulcorants dérivant de l'acide L-aspartique, de formule générale suivante :

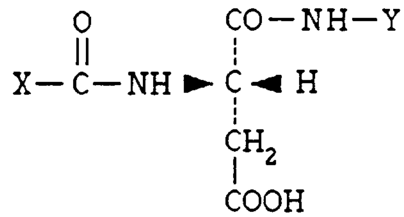

dans laquelle X est $CF_3$ ou $CCl_3$ et dans laquelle Y est 4-CN-$C_6H_4$, 4-Cl-$C_6H_4$, 4-Br-$C_6H_4$, 4-F-$C_6H_4$ ou $C_6H_5$.

Tinti *et al.* ont montré que le résidu L-aspartyle des composés précédents peut être remplacé, souvent avantageusement, par son homologue supérieur, le résidu L-glutamyle (Naturwissenschaften, 1981, 68, 143).

Tsuchiya *et al.* ont décrit, comme agents édulcorants, des composés de formule :

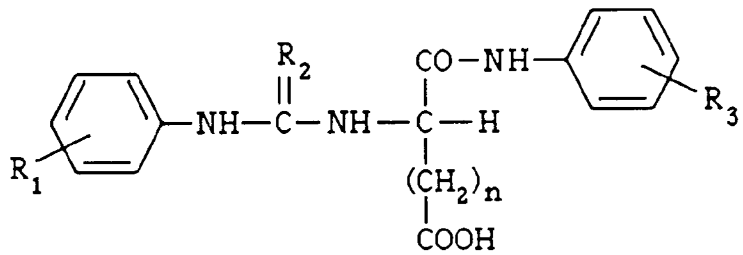

dans laquelle $R_1$ et $R_3$ sont H, les halogènes, CN, $NO_2$, R′ (R′ étant un groupe $C_1$-$C_6$ alkyle), COOR″ (R″ étant un groupe $C_1$-$C_4$ alkyle), R″CO, halogénométhyle, R″O, CONHR″, $SO_2R$″ ou SOR″, dans laquelle $R_2$ est O ou S, et dans laquelle n est 0, 1 ou 2 (JP-A-86-260052),
ou de formule :

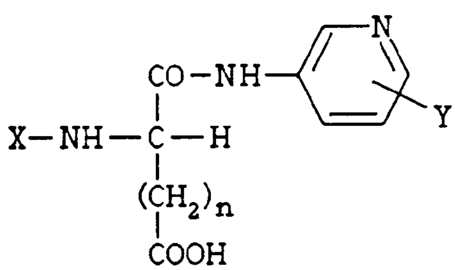

dans laquelle X est $CF_3CO$ ou $CCl_3CO$, dans laquelle Y est CN ou $NO_2$ et dans laquelle n est 1 ou 2 (JP-A-87-132863),
ou de formule :

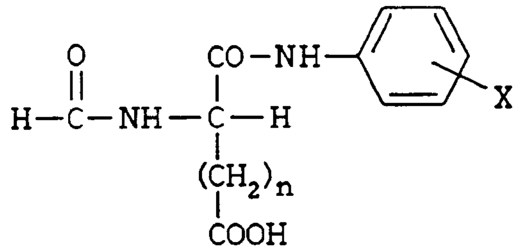

dans laquelle X est CN ou $NO_2$ et dans laquelle n est 1 ou 2 (JP-A-87-132847), ou de formule :

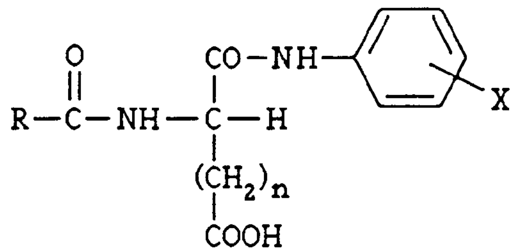

dans laquelle X est CN ou $NO_2$, dans laquelle R est H, $C_1$-$C_{10}$ alkyle ou un groupe aromatique alkoxy ou aryloxy et dans laquelle n est 1 ou 2 (JP-A-87-252754).

Les composés décrits dans l'art antérieur sont donc tous des dérivés N-formylés, N-acylés, N-carbamoylés ou N-thiocarbamoylés des acides aspartique ou glutamique alpha monoamides. Ils ont donc un groupe carbonyle C=O (ou thiocarbonyle C=S) directement fixé sur le groupe alpha-amino des résidus aspartyle ou glutamyle.

L'inconvénient majeur de ces dérivés N-formylés, N-acylés, N-carbamoylés ou N-thiocarbamoylés pour leur utilisation comme agent édulcorant réside dans leur faible stabilité en solution aqueuse (dans les conditions donc d'utilisation habituelle des édulcorants de synthèse) par suite d'une facilité d'hydrolyse de leur partie N-formylée, N-acylée, N-carbamoylée ou N-thiocarbamoylée avec réduction concomitante du pouvoir édulcorant, ce qui limite considérablement leur application industrielle éventuelle.

Les agents édulcorants de la présente invention ont la formule générale suivante :

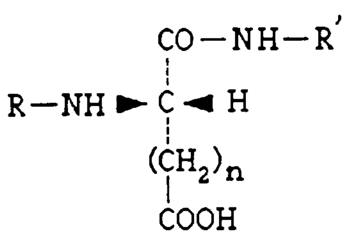

et sont caractérisés en ce que :

- R est un groupe hydrocarboné de 5 à 13 atomes de carbone, saturé ou insaturé, acyclique, cyclique ou mixte ;
- R' est un groupe 4-cyanophényle, 2-cyanopyrid-5-yle ou 2-cyanopyrimidin-5-yle ;
- n est égal à 1 ou 2.

Avantageusement :

- R est choisi dans le groupe constitué par :
  $C_5$-$C_{13}$ alk(én)yl normal,
  $C_5$-$C_{13}$ alk(én)yl ramifié,
  $C_5$-$C_{13}$ alk(én)yl cycloalk(én)yl,
  $C_6$-$C_{13}$ alk(én)yl cycloalk(én)yl alkyl,

$C_8$-$C_{13}$ alk(én)yl bicycloalk(én)yl ou
$C_8$-$C_{13}$ alk(én)yl bicycloalk(én)yl fusionné.

Dans une forme de réalisation préférée, R est choisi dans le groupe constitué par :

$CH_3(CH_2)_4$,
$CH_3(CH_2)_5$,
$CH_3(CH_2)_6$,
$CH_3(CH_2)_7$,
$CH_3(CH_2)_2CH{=}CHCH_2$,
$CH_3CH{=}CHCH{=}CHCH_2$,
$CH_3(CH_2)_2CH(CH_3)CH_2$,
$CH_3(CH_2)_3CH(CH_3)CH_2$,
$CH_3(CH_2)_4CH(CH_3)CH_2$,
$(CH_3)_2CH(CH_2)_4$,
$(CH_3)_2CH(CH_2)_5$,
c-$C_6H_{11}CH_2$,
c-$C_6H_{11}CH(CH_3)CH_2$,
c-$C_6H_{11}(CH_2)_3$,
c-$C_6H_{11}CH_2CH(CH_3)CH_2$,
$C_6H_5CH_2$,
$C_6H_5(CH_2)_2$,
$C_6H_5(CH_2)_3$,
$C_6H_5CH(CH_3)CH_2$,
$C_6H_5CH_2CH(CH_3)CH_2$,
$[(CH_3)_3C]_2CH(CH_2)_3$,
$[(CH_3)_3C]_2CHCH_2CH(CH_3)CH_2$,
(c-$C_3H_5)_2CH(CH_2)_3$,
(c-$C_3H_5)_2CHCH_2CH(CH_3)CH_2$,
$C_6H_5CH{=}CHCH_2$,
c-$C_6H_{11}CH{=}CHCH_2$,
1-naphtylpropyl,
1-perhydronaphtylpropyl,
1-indénylpropyl,
1-indanylpropyl,
1-perhydroindénylpropyl,
2,2,5,5-tétraméthylcyclopentylpropyl,
2-méthyl-3-indénylpropyl,
2-méthyl-3-indanylpropyl ou
2-méthyl-3-(2,2,5,5-tétraméthylcyclopentyl)-propyl.

En d'autres termes, les agents édulcorants de la présente invention se distinguent des composés proposés dans l'état de la technique rappelé dans le préambule en ce qu'ils sont des dérivés N-hydrocarbonés des acides L-aspartique ou L-glutamique sous forme de N-aryl ou N-hétéroaryl alpha-amides. Ils se distinguent donc fondamentalement sur le plan chimique des dérivés N-formylés, N-acylés, N-carbamoylés ou N-thiocarbamoylés décrits dans l'art antérieur.

De plus, à la différence de certains des composés cités dans l'art antérieur, les dérivés N-hydrocarbonés des acides aspartique et glutamique de l'invention n'ont une activité édulcorante que dans leur configuration L (comme cela ressort de la formule générale dans laquelle les liaisons portées par le carbone asymétrique sont représentées par un triangle pour les liaisons au-dessus du plan et par un trait discontinu pour les liaisons au-dessous du plan).

Les composés de l'invention constituent les premiers dérivés connus d'alpha amino acides N-hydrocarbonés à caractère édulcorant. L'obtention d'une activité édulcorante chez ces dérivés N-hydrocarbonés est d'autant plus inattendu que, comme on le sait, toute modification, même légère, de la structure moléculaire d'un agent édulcorant peut entraîner une suppression de l'activité édulcorante, et ce d'autant que les relations entre la structure et l'activité édulcorante sont imprévisibles (voir par exemple M. G. J. BEETS, Structure-Activity Relationships in Human Chemoreception, Applied Science Pub., London, 1978, pp. 259-362, H. VAN DER WEL, A. VAN DER HEIDJEN, H.G. PEER, Food Reviews International, 1987, 3, 193-268.).

En outre, ce remplacement des groupes N-formyle, N-acyle, N-carbamoyle ou N-thiocarbamoyle par un groupe N-hydrocarboné a pour effet d'augmenter fortement l'activité édulcorante puisqu'il est possible d'obtenir, avec certains des composés de la présente invention, une activité édulcorante qui est jusqu'à 7000 fois

plus élevée que celle du saccharose, c'est-à-dire au moins 2 à 3 fois plus élevée que celle des composés N-acylés les plus puissants décrits dans l'art antérieur précédemment cité. Cette augmentation spectaculaire de l'activité édulcorante a le double avantage d'abaisser les coûts d'utilisation de ces composés mais aussi les risques toxicologiques en diminuant les quantités nécessaires pour édulcorer les préparations alimentaires.

Le remplacement des groupes N-formyle, N-acyle, N-carbamoyle ou N-thiocarbamoyle par un groupe N-hydrocarboné améliore aussi, de manière inattendue, la stabilité en solution de ces composés, ce qui devrait permettre de pouvoir les utiliser avec succès dans la préparation des boissons ou des aliments, contrairement aux dérivés N-formylés, N-acylés, N-carbamoylés ou N-thiocarbamoylés. En effet, dans les conditions d'utilisation à pH acide, ce qui est généralement le cas des boissons gazeuses qui représentent la part la plus importante du marché des édulcorants, les composés de l'art antérieur cités dans le préambule ont une moindre stabilité que celle des composés de la présente invention. C'est ainsi par exemple qu'on a pu constater, par vieillissement accéléré (chauffage prolongé à 70 °C d'une solution à pH 3), que l'acide (S)-3-(4-cyanophénylcarbamoyl)-3-(phénylméthylamino)propanoïque décrit dans les Exemples est environ 3 fois plus stable que l'acide N-trifluoroacétyl-alpha-L-aspartyl-4-cyanoanilide décrit dans le brevet US-A-3.725.453.

Les composés de la présente invention peuvent être préparés par des méthodes variées déjà décrites dans la littérature.

L'une des méthodes préférée consiste à condenser le précurseur aminé de formule générale suivante:

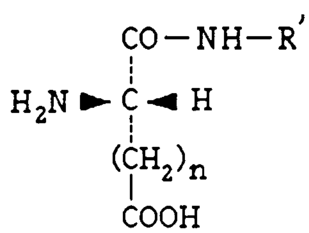

avec un aldéhyde ou une cétone $C_5$-$C_{13}$ hydrocarboné, l'imine résultante étant réduite par le cyanoborohydrure de sodium suivant la technique de N-monoalkylation réductive des alpha-amino acides décrite par Ohfune *et al.* (Chem. Letters, 1984, 441-444). La réaction est effectuée à température ambiante dans le méthanol.

Les agents édulcorants de l'invention peuvent aussi être salifiés par des acides ou des bases inorganiques ou organiques physiologiquement acceptables, ce qui a pour effet d'améliorer leur solubilité et/ou leur stabilité. Avantageusement, ces composés sont salifiés sous forme d'hydrochlorure ou de sels de sodium, potassium, ammonium, calcium ou magnésium.

La purification des composés de l'invention, sous leur forme acide ou salifiée, est réalisée selon les techniques standards telles que la recristallisation ou la chromatographie. Leur structure et leur pureté ont été contrôlées par les techniques classiques (chromatographie sur couche mince, chromatographie liquide haute performance, spectrométrie infra-rouge, résonance magnétique nucléaire, analyse élémentaire).

Les agents édulcorants de la présente invention peuvent être ajoutés à tout produit comestible dans lequel on désire apporter un goût sucré, à condition de les ajouter en proportions suffisantes pour atteindre le niveau d'édulcoration désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau d'édulcoration désiré. Toute personne qualifiée peut facilement déterminer la proportion optimale d'agent édulcorant qui doit être employée pour l'obtention d'un produit comestible en réalisant des analyses sensorielles de routine. Les agents édulcorants de la présente invention sont, en général, ajoutés aux produits comestibles dans des proportions allant, suivant le pouvoir édulcorant du composé, de 50 mg à 500 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

Les agents édulcorants de la présente invention peuvent être ajoutés sous forme pure aux produits comestibles pour leur communiquer un goût sucré. Toutefois, par suite de leur pouvoir sucrant élevé, ils sont généralement mélangés à un support ("carrier") ou à un agent de charge ("bulking agent") approprié.

Le pouvoir édulcorant des composés décrits dans les exemples a été évalué par un groupe de huit goûteurs expérimentés. Pour cela, les composés, en solution aqueuse à des concentrations variables, sont comparés, sur le plan gustatif, à une solution témoin de saccharose à 2 %. Le pouvoir édulcorant du composé, testé par rapport au saccharose, correspond alors au rapport pondéral qui existe entre le composé et le saccharose à égale intensité édulcorante, c'est-à-dire quand les saveurs sucrées de la solution du composé testé et de la

solution témoin de saccharose sont considérées, par une majorité de goûteurs, avoir la même intensité édulcorante.

La manière dont l'invention peut être réalisée et les avantages qui en découlent ressortiront mieux des 25 exemples de réalisation qui suivent.

EXEMPLES :

Synthèse de l'acide (S)-3-(4-cyanophénylcarbamoyl)-3-(phénylméthylamino)propanoïque, de formule :

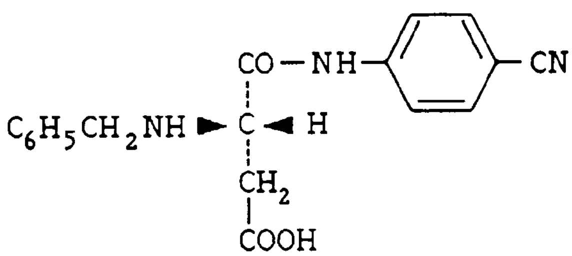

Pour préparer ce composé, 1,59 g (0,015 mole) de benzaldéhyde est ajouté à un mélange de 2,35 g (0,01 mole) d'alpha-L-aspartyl-4-cyanoanilide et de 0,62 g (0,01 mole) de cyanoborohydrure de sodium. La solution est agitée durant 3 jours à 20°C puis est filtrée avant d'être concentrée à sec sous vide. Le résidu est dissous dans une solution de carbonate de sodium à 3 % (30 $cm^3$). La solution obtenue est lavée par l'éther éthylique (3 x 50 $cm^3$) avant d'être acidifiée à pH ≈ 5 - 6 par HCl 3N, ce qui permet d'obtenir 0,38 g d'acide (S)-3-(4-cyanophénylcarbamoyl)-3-(phénylméthylamino)propanoïque (rendement 12 % ; point de fusion 178°C).

Le pouvoir édulcorant de ce composé correspond approximativement, sur une base pondérale, à 1 000 (mille fois) celui du saccharose (par comparaison avec une solution de saccharose à 2 %).

Le tableau récapitulatif ci-dessous donne, à titre d'exemples, la liste de 25 composés selon l'invention obtenus suivant un protocole expérimental similaire à celui décrit ci-dessus.

Dans ce tableau sont également portés les rendements (Rdt) obtenus, les points de fusion (F) exprimés en degrés Celsius (°C) et le pouvoir édulcorant (établi par comparaison avec une solution de saccharose à 2 %).

EXEMPLES

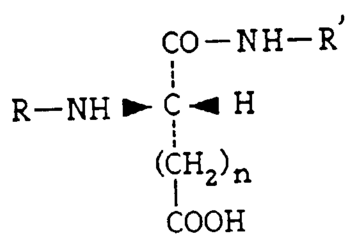

| R | R' | n | Rdt (%) | F (°C) | Pouvoir édulcorant |
|---|---|---|---|---|---|
| $CH_3(CH_2)_3CH_2$ | $4\text{-}CN\text{-}C_6H_4$ | 1 | 18 | 172 | 300 |
| $CH_3(CH_2)_4CH_2$ | $4\text{-}CN\text{-}C_6H_4$ | 1 | 56 | 140 | 600 |
| $CH_3(CH_2)_5CH_2$ | $4\text{-}CN\text{-}C_6H_4$ | 1 | 54 | 142 | 2 000 |
| $CH_3(CH_2)_6CH_2$ | $4\text{-}CN\text{-}C_6H_4$ | 1 | 49 | 140 | 400 |
| $(CH_3CH_2)_2CHCH_2$ | $4\text{-}CN\text{-}C_6H_4$ | 1 | 53 | 156 | 200 |
| $(CH_3)_2CHCH_2CH_2$ | $4\text{-}CN\text{-}C_6H_4$ | 1 | 17 | 187 | 100 |
| $c\text{-}C_6H_{11}CH_2$ | $4\text{-}CN\text{-}C_6H_4$ | 1 | 8 | 186 | 200 |
| $c\text{-}C_6H_{11}CH(CH_3)$ | $4\text{-}CN\text{-}C_6H_4$ | 1 | 41 | 184 | 200 |
| $C_6H_5CH_2$ | $4\text{-}CN\text{-}C_6H_4$ | 1 | 12 | 178 | 1 000 |
| $C_6H_5CH_2CH_2$ | $4\text{-}CN\text{-}C_6H_4$ | 1 | 44 | 179 | 300 |
| $CH_3(CH_2)_3CH_2$ | $4\text{-}CN\text{-}C_6H_4$ | 2 | 25 | 134 | 1 500 |
| $CH_3(CH_2)_4CH_2$ | $4\text{-}CN\text{-}C_6H_4$ | 2 | 36 | 142 | 5 000 |
| $CH_3(CH_2)_5CH_2$ | $4\text{-}CN\text{-}C_6H_4$ | 2 | 74 | 143 | 7 000 |
| $CH_3(CH_2)_6CH_2$ | $4\text{-}CN\text{-}C_6H_4$ | 2 | 76 | 135 | 2 000 |
| $(CH_3)_2CHCH_2CH_2$ | $4\text{-}CN\text{-}C_6H_4$ | 2 | 15 | 173 | 800 |
| $CH_3(CH_2)_2CH(CH_3)CH_2$ | $4\text{-}CN\text{-}C_6H_4$ | 2 | 63 | 127 | 5 000 |
| $CH_3(CH_2)_2CH{=}CHCH_2$ | $4\text{-}CN\text{-}C_6H_4$ | 2 | 50 | 138 | 3 000 |
| $CH_3CH{=}CHCH{=}CHCH_2$ | $4\text{-}CN\text{-}C_6H_4$ | 2 | 36 | 140 | 2 000 |

| R | R' | n | Rdt (%) | F (°C) | Pouvoir édulcorant |
|---|---|---|---|---|---|
| $C_6H_5CH_2$ | 4-CN-$C_6H_4$ | 2 | 16 | 157 | 20 |
| $C_6H_5CH{=}CHCH_2$ | 4-CN-$C_6H_4$ | 2 | 39 | 140 | 2 000 |
| $C_6H_5CH(CH_3)CH_2CH_2$ | 4-CN-$C_6H_4$ | 2 | 32 | 138 | 400 |
| c-$C_6H_{11}CH_2CH_2CH_2$ | 4-CN-$C_6H_4$ | 2 | 67 | 145 | 7 000 |
| $C_6H_5CH_2CH_2$ | 4-CN-$C_6H_4$ | 2 | 37 | 125 | 300 |
| $C_6H_5CH_2CH_2CH_2$ | 4-CN-$C_6H_4$ | 2 | 59 | 149 | 800 |
| $CH_3(CH_2)_5CH_2$ | 2-CN-5-pyrid-5-yl | 2 | 26 | 146 | 4 000 |

On constate que 12 des 25 composés décrits dans le tableau ci-dessus ont un pouvoir sucrant compris entre 1 000 et 7 000 fois celui du saccharose, ce qui représente, pour les composés les plus actifs, une intensité édulcorante 2 à 3 fois plus élevée que celle des composés de l'art antérieur précédemment cité.

## Revendications

1. Agents édulcorants de formule générale :

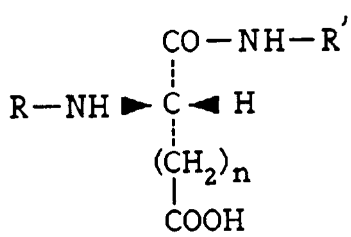

caractérisés en ce que :

– R est un groupe hydrocarboné ayant de 5 à 13 atomes de carbone, saturé ou insaturé, acyclique, cyclique ou mixte ;
– R' est un groupe 4-cyanophényle, 2-cyanopyrid-5-yle ou 2-cyanopyrimidin-5-yle ;
– n est égal à 1 ou à 2.

2. Agents édulcorants suivant la revendication 1 caractérisés en ce que R est choisi dans le groupe constitué par :

$C_5$-$C_{13}$ alk(én)yl normal,
$C_5$-$C_{13}$ alk(én)yl ramifié,
$C_5$-$C_{13}$ alk(én)yl cycloalk(én)yl,
$C_6$-$C_{13}$ alk(én)yl cycloalk(én)yl alkyl,
$C_8$-$C_{13}$ alk(én)yl bicycloalk(én)yl ou
$C_8$-$C_{13}$ alk(én)yl bicycloalk(én)yl fusionné.

3. Agents édulcorants suivant les revendications 1 et 2 caractérisés en ce que R est choisi dans le groupe constitué par :

$CH_3(CH_2)_4$,

$CH_3(CH_5)_5$,
$CH_3(CH_2)_6$,
$CH_3(CH_2)_7$,
$CH_3(CH_2)_2CH{=}CHCH_2$,
$CH_3CH{=}CHCH{=}CHCH_2$,
$CH_3(CH_2)_2CH(CH_3)CH_2$,
$CH_3(CH_2)_3CH(CH_3)CH_2$,
$CH_3(CH_2)_4CH(CH_3)CH_2$,
$(CH_3)_2CH(CH_2)_4$,
$(CH_3)_2CH(CH_2)_5$,
$c\text{-}C_6H_{11}CH_2$,
$c\text{-}C_6H_{11}CH(CH_3)CH_2$,
$c\text{-}C_6H_{11}(CH_2)_3$,
$c\text{-}C_6H_{11}CH_2CH(CH_3)CH_2$,
$C_6H_5CH_2$,
$C_6H_5(CH_2)_2$,
$C_6H_5(CH_2)_3$,
$C_6H_5CH(CH_3)CH_2$,
$C_6H_5CH_2CH(CH_3)CH_2$,
$[(CH_3)_3C]_2CH(CH_2)_3$,
$[(CH_3)_3C]_2CHCH_2CH(CH_3)CH_2$,
$(c\text{-}C_3H_5)_2CH(CH_2)_3$,
$(c\text{-}C_3H_5)_2CHCH_2CH(CH_3)CH_2$,
$C_6H_5CH{=}CHCH_2$,
$c\text{-}C_6H_{11}CH{=}CHCH_2$,
1-naphtylpropyl,
1-perhydronaphtylpropyl,
1-indénylpropyl,
1-indanylpropyl,
1-perhydroindénylpropyl,
2,2,5,5-tétraméthylcyclopentylpropyl,
2-méthyl-3-indénylpropyl,
2-méthyl-3-indanylpropyl ou
2-méthyl-3-(2,2,5,5-tétraméthylcyclopentyl)-propyl.

## Patentansprüche

1. Süßstoffe der allgemeinen Formel

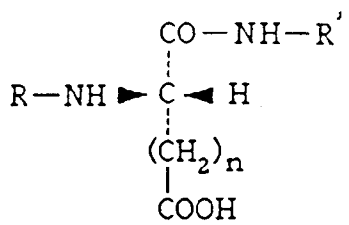

dadurch gekennzeichnet, daß:
- R eine gesättigte oder ungesättigte, acyclische, cyclische oder gemischte Kohlenwasserstoffgruppe mit 5 bis 13 Kohlenstoffatomen ist;
- R′ eine 4-Cyanophenyl-, 2-Cyanopyrid-5-yl- oder 2-Cyanopyrimidin-5-ylgruppe ist;
- n für 1 oder 2 steht.

2. Süßstoffe nach Anspruch 1, dadurch gekennzeichnewt, daß R ausgewählt ist aus der Gruppe bestehend aus:

$C_5$-$C_{13}$ Alk (en ) yl normal,
$C_5$-$C_{13}$ Alk (en) yl ramifiziert,

$C_5$-$C_{13}$ Alk (en) yl-cycloalk (en) yl,
$C_6$-$C_{13}$ Alk (en) yl -cycloalk (en) yl-alkyl,
$C_8$-$C_{13}$ Alk (en) yl-bicycloalk (en) yl oder
$C_8$-$C_{13}$ Alk (en) yl-bicycloalk (en) yl gemischt.

3. Süßstoffe nach den Ansprüchen 1 und 2, dadurch gekennzeichnet, daß R ausgewählt ist aus der Gruppe bestehend aus:

$CH_3(CH_2)_4$,
$CH_3(CH_2)_5$,
$CH_3(CH_2)_6$,
$CH_3(CH_2)_7$,
$CH_3(CH_2)_2CH{=}CHCH_2$,
$CH_3CH{=}CHCH{=}CHCH_2$,
$CH_3(CH_2)_2CH(CH_3)CH_2$,
$CH_3(CH_2)_3CH(CH_3)CH_2$,
$CH_3(CH_2)_4CH(CH_3)CH_2$,
$(CH_3)_2CH(CH_2)_4$,
$(CH_3)_2CH(CH_2)_5$,
c-$C_6H_{11}CH_2$ ,
c-$C_6H_{11}CH(CH_3)CH_2$,
c-$C_6H_{11}(CH_2)_3$,
c-$C_6H_{11}CH_2CH(CH_3)CH_2$,
$C_6H_5CH_2$,
$C_6H_5(CH_2)_2$,
$C_6H_5(CH_2)_3$,
$C_6H_5CH(CH_3)CH_2$,
$C_6H_5CH_2CH(CH_3)CH_2$,
$[(CH_3)_3C]_2CH(CH_2)_3$,
$[(CH_3)_3C]_2CHCH_2CH(CH_3)CH_2$,
(c-$C_3H_5)_2CH(CH_2)_3$,
(c-$C_3H_5)_2CHCH_2CH(CH_3)CH_2$,
$C_6H_5CH{=}CHCH_2$,
C-$C_6H_{11}CH{=}CHCH_2$,
1-Naphthylpropyl,
1-Perhydronanhthylpropyl,
1-Indenylpropyl,
1-Indanylpropyl,
1-Perhydroindenylpropyl,
2,2,5,5-Tetramethylcyclopentylpropyl,
2-Methyl-3-indenylpropyl,
2-Methyl-3-indanylpropyl oder
2-Methyl-3-(2,2,5,5-tetramethylcyclopentyl)-propyl.

## Claims

1. Sweetening agents of the general formula

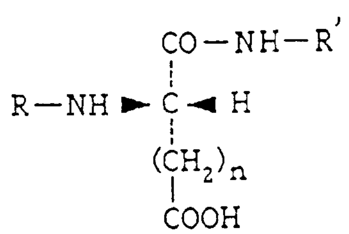

characterized in that :

– R is a saturated or unsaturated, acyclic, cyclic or mixed hydrocarbon group having 5 to 13 carbon atoms;

– R′ is a 4-cyanophenyl, 2-cyanopyrid-5-yl or 2-cyanopyrimidin-5-yl group; and
– n is 1 or 2.

2. Sweetening agents according to claim 1, characterized in that R is selected from the group consisting of:

normal alk(en)yl $C_5$-$C_{13}$,
branched alk(en)yl $C_5$-$C_{13}$,
cycloalk(en)yl alk(en)yl $C_5$-$C_{13}$,
alkyl cycloalk(en)yl alk(en)yl $C_6$-$C_{13}$,
bicycloalk(en)yl alk(en)yl $C_8$-$C_{13}$, or
fused bicycloalk(en)yl alk(en)yl $C_8$-$C_{13}$.

3. Sweetening agents according to claims 1 and 2, characterized in that R is selected from the group consisting of:

$CH_3(CH_2)_4$,
$CH_3(CH_2)_5$,
$CH_3(CH_2)_6$,
$CH_3(CH_2)_7$,
$CH_3(CH_2)_2CH{=}CHCH_2$,
$CH_3CH{=}CHCH{=}CHCH_2$,
$CH_3(CH_2)_2CH(CH_3)CH_2$,
$CH_3(CH_2)_3CH(CH_3)CH_2$,
$CH_3(CH_2)_4CH(CH_3)CH_2$,
$(CH_3)_2CH(CH_2)_4$,
$(CH_3)_2CH(CH_2)_5$,
c-$C_6H_{11}CH_2$,
c-$C_6H_{11}CH(CH_3)CH_2$,
c-$C_6H_{11}(CH_2)_3$,
c-$C_6H_{11}CH_2CH(CH_3)CH_2$,
$C_6H_5CH_2$,
$C_6H_5(CH_2)_2$,
$C_6H_5(CH_2)_3$,
$C_6H_5CH(CH_3)CH_2$,
$C_6H_5CH_2CH(CH_2)CH_2$,
$[(CH_3)_3C]_2CH(CH_2)_3$,
$[(CH_3)_3C]_2CHCH_2CH(CH_3)CH_2$,
(c-$C_3H_5)_2CH(CH_2)_3$,
(c-$C_3H_5)_2CHCH_2CH(CH_3)CH_2$,
$C_6H_5CH{=}CHCH_2$,
c-$C_6H_{11}CH{=}CHCH_2$,
1-naphthylpropyl,
1-perhydronaphthylpropyl,
1-indenylpropyl,
1-indanylpropyl,
1-perhydroindenylpropyl,
2,2,5,5-tetramethylcyclopentylpropyl,
2-methyl-3-indenylpropyl,
2-methyl-3-indanylpropyl, or
2-methyl-3-(2,2,5,5-tetramethylcyclopentyl)-propyl.